# EUROPEAN PATENT SPECIFICATION

(11) **EP 1 407 766 B1**
(45) Date of publication and mention of the grant of the patent: **01.07.2009**
(21) Application number: 03386023.0
(22) Date of filing: 03.10.2003
(51) Int. Cl.: A61K 9/16, A61K 9/20, A61K 9/00, A61K 31/663

(54) **Pharmaceutical compositions that contain a salt of alendronic acid**
Pharmazeutische Zusammensetzungen mit einem Salz von Alendronsäure
Compositions pharmaceutiques contenant un sel d'acide alendronique

(30) Priority: 07.10.2002 GR 2002100436
(43) Date of publication of application: 14.04.2004
(73) Proprietor: Verisfield (UK) Ltd., 115 28 Athens (GR)
(72) Inventor: Pantazopoulou, Myrsini, Athens (GR)

(56) References cited:
- WO-A-01/85176
- WO-A-02/03963
- US-A- 5 462 932

## Description

### Background of the Invention

International patent publication WO-A-01 85176, discloses solid oral pharmaceutical compositions containing alendronic acid as monosodium trihydrate and the use of specific excipients such as mannitol, magnesium stearate, crosslinked polyvinylpyrrolidone, polyvinylpyrrolidone, sodium lauryl sulphate and potato starch in order to produce tablets and capsules using wet granulation technique. Said formulation results in a stable final product, which by passes the unfavorable effect of Maillard reaction due to the use of lactose as a diluent in former patent applications. Lactose, used in such formulations, reacts with the amino group of alendronic acid giving degradation products that reduce the effectiveness of the drug. Other patents (WO-A-02 03963) make use of a combination of diluting agents such as mannitol microcrystalline cellulose, calcium hydrogen phosphate or modified starches in order to produce oral solid pharmaceutical preparations using direct compression. In this case the inferior compression characteristics of mannitol along with the hygroscopicity of microcrystalline cellulose and modified starches and the alkalinity of calcium hydrogen phosphate may affect the finished product characteristics and stability. Patent publication US-A-5 462 932, discloses a formulation for preparing oral liquid forms of Alendronic Acid for those who have difficulty in swallowing. The formulation contains a pH adjuster and a complexing agent for preventing the formation of insoluble complexes. The said patent discloses also the use of saccharin and sucrose as sweetening and flavoring agents.

### Description

The present invention relates to new pharmaceutical compositions that contain alendronic acid in the form of its salts and are used for treating and preventing osteoporosis.

The salts of alendronic acid, anhydrous or hydrous, may be salts of ammonium, salts of potassium, salts of aminoacids, salts of calcium, salts of magnesium or salts of organic bases.

In the present invention, alendronic acid (4-amino-1-hydroxybutylidene-1,1 bisphosphonic acid) in the form of a sodium salt trihydrate which has been formulated in a new manner, achieving products with perfect pharmaco-technical characteristics, is preferred.

Various methods and techniques are used for preparing powders or granules, the most important of which are the direct compression of powders and wet granulation.

Applying the method of direct compression of powders the difficulty, of course, with fine powders is that they often refuse to flow and give even die filling. Where the powders flow satisfactorily, the large quantities of air entrapped within them often present a further problem, leading to weight irregularities. Furthermore, certain powders have little ability to cohere and tend to return to their original condition after shaping, resulting to the subsequent breakage of the tablets.

By employing the technique of wet granulation, the binding agent prevents the tendency to break and decompose. Furthermore, as wet granulation occurs in the manufacturing area of the finished product, the size of the granules can be adjusted according to the requirements of the finished product.

Alendronic acid, as all the bisphosphonic acids is sensitive to moisture and temperature. By direct powder compression, the moisture of the excipients used cannot be fully adjusted, as these are used as received by their respective producers and as the acceptable moisture range for excipients is very wide.

Oppositely, by employing the method of wet granulation, it is possible to have complete control of the final residual moisture, during the stage of drying of the granules.

The present invention employs the method of wet granulation using purified water for producing mixtures used for the preparation of tablets, capsules or for filling sachets with granules or powders.

The composition of the mixtures includes:
- Active ingredient
- Binding agents
- Disintegrating agents
- Diluting agents
- Lubricating agents
- Flavouring substances
- Sweetening substances

The particular invention uses the following ingedients for each pharmaceutical form.

### 1. Tablets and capsules

a) Alendronic Acid monosodium salt trihydrate, as an active ingredient
b) Mannitol as diluting agent
c) Pregelatinised Starch, as binding agent
d) Sodium Starch Glycolate, as disintegrating agent
e) Talc or Magnesium Stearate as lubricating agent

### Pharmaceutical compositions for commercial use

| Components | Content w/w |
|---|---|
| Alendronic acid monosodium salt trihydrate | between 3.5% and 24.7% |
| Mannitol | between 39.3% and 30.6% |
| Pregelatinised starch | between 49.1% and 39.1% |
| Sodium starch glycolate | between 6.9% and 4.8% |
| Lubricating agent | between 1.2% to 0.8% |

### 2. Sachets with granules or powders

a) Alendronic Acid monosodium salt trihydrate, as an active ingredient
b) Mannitol as diluting agent
c) Sugar as sweetening agent
d) Saccharin sodium as sweetening agent
e) Maltodextrin as binding agent
f) Flavour as taste improving agent

### Pharmaceutical compositions for commercial use

| Components | Content w/w |
|---|---|
| Alendronic acid monosodium salt trihydrate | between 065% and 9.14% |
| Mannitol | between 49.05% and 40.56% |
| Sugar | 45.00% |
| Saccharin sodium | 0.50% |
| Maltodextrin | 0.80% |
| Flavour | 4.00% |

### Method of production of tablets or capsules

The compositions of the present invention are prepared by employing the method of wet granulation, using traditional methodologies of mixing, granulation, drying ,tabletting and filling of capsules.

The active ingredient and the auxiliary components (excipients) are sieved separately.

The following are added to a powder mixer:
a) all the required quantity of mannitol, as diluting agent
b) all the required quantity of sodium starch glycolate as an disintegrating/breakage agent
c) all the required quantity of pregelatinised starch as a binding agent

The above are mixed for 15 minutes at a speed of rotation of the mixer of 20 RPM.

In the sequel, the mixture of powders is transferred to a granulation unit and brought to granule size by adding purified water sprayed to the granulation mixer.

The paste that derives is forced to pass through a suitable aperture (10 Mesh) sieve.

The wet granules that derive are dried by employing drying techniques in microwaves, fluidized bed dryers, discs with receptors, to a residual moisture level of 1 to 2% The dried granules are ground for producing a mixture with a suitable particle size using a suitable aperture (20 Mesh) sieve.

The granulated mixture is weighted and transferred to a powder mixture. In the sequel, the required quantity of alendronic acid monosodium salt trihydrate is added and the mixture is mixed for 20 minutes at a mixer speed of 20 RPM. Finally,the lubricating agent is added to the powder mixture and stirring continues for an additional five minutes period, keeping the speed of rotation of the powder mixer constant.

The powder mixture, after lubrication can be used to make tablets by compressing the mixture in a tabletting machine using the suitable punches and dies, or to make capsules, by filling empty hard gelatine capsules with the mixture.

The weight of the tablets and capsules is calculated so that the necessary quantity of active ingredient is provided per dosage unit (tablet or capsule).

### Method of production of mixtures of granules or powders for filling sachets

The compositions of the present invention are prepared by employing the method of wet granulation, using traditional methodologies of mixing, granulation, drying and filling of sachets.

The active compound and the auxiliary components (excipients) are sieved separately.

The following are added to a powder mixer of suitable dimensions:
a) all the required quantity of mannitol, as diluting agent
b) all the required quantities of sugar and saccharin sodium as sweetening agents
c) all the required quantity of maltodextrine as a binding agent

The above are mixed for 15 minutes at a speed of rotation of the mixer of 20 RPM.

In the sequel, the mixture of powders is transferred to a granulation unit and brought to granule size by adding purified water sprayed to the granulation mixer.

The paste that derives is forced to pass through a suitable aperture (5 Mesh) sieve.

The wet granules that derive are dried by employing drying techniques in microwaves, fluidized bed dryers, discs with receptors, to a residual moisture level of 1 to 2% The dried granules are ground for producing a mixture with a suitable particle size using a suitable aperture (15 Mesh for granules or 60 Mesh for powders) sieve.

The granulated mixture is weighted and transferred to a powder mixture. In the sequel, the required quantity of Alendronic acid monosodium salt trihydrate, as the active compound, and the flavour are added as well, and the mixture is mixed for 20 minutes at a mixer speed of 20 RPM.

The powder mixture, after completion of mixing is transferred to the sachet filling machine where the aluminum lined sachets are filled.

The weight of the granules or powders per sachet is calculated so that the necessary quantity of active compound is provided.

### Examples of compositions of tablets and/or capsules

### Example 1

Composition of tablets and/or capsules with a 5 mg active ingredients content

| | | |
|---|---|---|
| Alendronic acid monosodium salt trihydrate corresponding to alendronic acid | Active ingredient | 6.52 mg (5.00 mg) |
| Mannitol | Diluting agent | 72.78 mg |
| Pregelatinised starch | Binding agent | 90.91 mg |
| Sodium starch glycolate | Disintegrating agent | 12.79 mg |
| Talc | Lubricating agent | 2.00 mg |
| Weight of tablet or of contents of capsule | | 185 mg |

The method of preparation of the tablets or ofthe filling mixtureof the capsules is as described in the respective paragraph.

### Example 2

Composition of tablets and/or capsules with a 10 mg active ingredient content

| | | |
|---|---|---|
| Alendronic acid monosodium salt trihydrate corresponding to alendronic acid | Active ingredient | 13.05 mg (10.00 mg) |
| Mannitol | Diluting agent | 66.25 mg |
| Pregelatinised starch | Binding agent | 90.91 mg |
| Sodium starch glycolate | Disintegrating agent | 12.79 mg |
| Talc | Lubricating agent | 2.00 mg |
| Weight of tablet or of contents of capsule | | 185 mg |

The method of preparation of the tablets or ofthe filling mixtureof the capsules is as described in the respective paragraph.

### Example 3

Composition of tablets and/or capsules with a 20 mg active inqredient content

| | | |
|---|---|---|
| Alendronic acid monosodium salt trihydrate corresponding to alendronic acid | Active ingredient | 26.10 mg (20.00 mg) |
| Mannitol | Diluting agent | 53.20 mg |
| Pregelatinised starch | Binding agent | 90.91 mg |
| Sodium starch glycolate | Disintegrating agent | 12.79 mg |
| Talc | Lubricating agent | 2.00 mg |
| Weight of tablet or of contents of capsule | | 185 mg |

The method of preparation of the tablets or of the filling mixtureof the capsules is as described in the respective paragraph.

### Example 4

Composition of tablets and/or capsules with a 50 mg active ingredient content

| | | |
|---|---|---|
| Alendronic acid monosodium salt trihydrate corresponding to alendronic acid | Active ingredient | 65.25 mg (50.00 mg) |
| Mannitol | Diluting agent | 139.52 mg |
| Pre-gelatinised starch | Binding agent | 144.80 mg |
| Sodium starch glycolate | Disintegrating agent | 17.70 mg |
| Talc | Lubricating agent | 2.73 mg |
| Weight of tablet or of contents of capsule | | 370 mg |

The method of preparation of the tablets or of the filling mixtureof the capsules is as described in the respective paragraph.

### Example 5

Composition of tablets and/or capsules with a 70 mg active ingredient content

| | | |
|---|---|---|
| Alendronic acid monosodium salt tri-hydrate corresponding to alendronic acid | Active ingredient | 91.37 mg (70.00 mg) |
| Mannitol | Diluting agent | 113.40 mg |
| Pregelatinised starch | Binding agent | 144.80 mg |
| Sodium starch glycolate | Disintegrating agent | 17.70 mg |
| Talc | Lubricating agent | 2.73 mg |
| Weight of tablet or of contents of capsule | | 370 mg |

The method of preparation of the tablets or of the filling mixture of the capsules is as described in the respective paragraph.

### Example 6

Triple comparative Dissolution test was performed for:
- Samples of tablets with a 10 mg content of the active compound prepared according to the method described in the present invention
- Samples of capsules with a 10 mg content of the active ingredient prepared according to the method described in the present invention
- Samples of FOSAMAX tablets with a 10 mg content of the active compound bought from a commercial outlet (pharmacy).

Six units from each sample were used.

Water (900 ml) was used as the dissolution medium, the speed of rotation was set at 50 RPM and sampling and measurement of the samples was performed after 30 minutes of stirring.

All results (18 samples) were found within the specified limits (minimum acceptable solubility of 80%) and in any case the deviations of measurements of individual units were not higher than 3%.

### Examples of compositions of granules and/or powders for filling sachets

### Example 1

Composition of granules and/or powders with a 5 mg active ingredient content

| | | |
|---|---|---|
| Alendronic acid monosodium salt trihydrate corresponding to alendronic acid | Active ingredient | 6.52 mg (5.00 mg) |
| Mannitol | Diluting agent | 490.48 mg |
| Sugar | Sweetening agent | 450.00 mg |
| Saccharin Sodium | Sweetening agent | 5.00 mg |
| Maltodextrin | Binding agent | 8.00 mg |
| Flavour | Taste Improving Agent | 40.00 mg |
| Weight of granules or powder per sachet | | 1000 mg |

The method of preparation of the granules or powders for filling sachets is as described in the respective paragraph.

### Example 2

Composition of granules and/or powders with a 10 mg active ingredient content

| | | |
|---|---|---|
| Alendronic acid monosodium salt trihydrate corresponding to alendronic acid | Active ingredient | 13.05 mg (10.00 mg) |
| Mannitol | Diluting agent | 483.95 mg |
| Sugar | Sweetening agent | 450.00 mg |
| Saccharin Sodium | Sweetening agent | 5.00 mg |
| Maltodextrin | Binding agent | 8.00 mg |
| Flavour | Taste Improving Agent | 40.00 mg |
| Weight of granules or powder per sachet | | 1000 mg |

The method of preparation of the granules or powders for filling sachets is as described in the respective paragraph.

### Example 3

Composition of granules and/or powders with a 20 mg active ingedient content

| | | |
|---|---|---|
| Alendronic acid monosodium salt trihydrate corresponding to alendronic acid | Active Compound | 26.10 mg (20.00 mg) |
| Mannitol | Diluting agent | 470.90 mg |
| Sugar | Sweetening agent | 450.00 mg |
| Saccharin Sodium | Sweetening agent | 5.00 mg |
| Maltodextrin | Binding agent | 8.00 mg |
| Flavour | Taste Improving Agent | 40.00 mg |
| Weight of granules or powder per sachet | | 1000 mg |

The method of preparation of the granules or powders for filling sachets is as described in the respective paragraph.

### Example 4

Composition of granules and/or powders with a 50 mg active ingredient content

| | | |
|---|---|---|
| Alendronic acid monosodium salt trihydrate corresponding to alendronic acid | Active ingredient | 65.25 mg (50.00 mg) |
| Mannitol | Diluting agent | 431.75 mg |
| Sugar | Sweetening agent | 450.00 mg |
| Saccharin Sodium | Sweetening agent | 5.00 mg |
| Maltodextrin | Binding agent | 8.00 mg |
| Flavour | Taste Improving Agent | 40.00 mg |
| Weight of granules or powder per sachet | | 1000 mg |

The method of preparation of the granules or powders for filling sachets is as described in the respective paragraph.

### Example 5

Composition of granules and/or powders with a 50 mg active ingredient content

| | | |
|---|---|---|
| Alendronic acid monosodium salt trihydrate corresponding to alendronic acid | Active ingredient | 91.37 mg (70.00 mg) |
| Mannitol | Diluting agent | 405.63 mg |
| Sugar | Sweetening agent | 450.00 mg |
| Saccharin Sodium | Sweetening agent | 5.00 mg |
| Maltodextrin | Binding agent | 8.00 mg |
| Flavour | Taste Improving Agent | 40.00 mg |
| Weight of granules or powder per sachet | | 1000 mg |

The method of preparation of the granules or powders for filling sachets is as described in the respective paragraph.

## Claims

1. A pharmaceutical composition for oral administration, which contains Alendronic Acid in the form of monosodium salt trihydrate and a pharmaceutically acceptable carrier, which is composed by a specific, stable combination of a diluent and a binder using Mannitol as a diluting agent and Pregelatinised Starch or Maltodextrin as a binding agent and
depending on the pharmaceutical form, Sodium Starch Glycolate as a disintegrating agent, Talc or Magnesium stearate as a lubricating agent, Sugar and Saccharin Sodium as sweetening agents and Flavour as taste improving agent.

2. A pharmaceutical composition according to Claim 1, for solid, orally administrated forms such as tablets and capsules that contains between 3.5% and 29.7% w/w Alendronic Acid monosodium salt trihydrate, between 69.7%and 88.5% w/w of the stable combination that consist of Mannitol as diluting agent and Pregelatinised Starch as binding agent, and between 5.6% and 8.0% w/w excipients, that consist of Sodium Starch Glycolate as dirintegating agent and Talc or Magnesium Stearate as lubricating agent.

3. A pharmaceutical composition according to Claim 2, that contains: Alendronic Acid monosodium salt trihydrate, between 3.5% and 24.7% w/w and Mannitol between 30.6% and 39.3% w/w.

4. A pharmaceutical composition according to Claim 2, that contains: Pregelatinised Starch between 39.1% and 49.1% w/w, Sodium Starch Glycolate between 4.8% and 6.9% w/w, Talc or Magnesium Stearate between 0.8% and 1.2% w/w.

5. A tablet or capsule including the pharmaceutical composition of claim 2.

6. A pharmaceutical composition, according to claim 1, for forms provided orally after reconstitution such as granules or powders for oral suspensions that contains, Alendronic Acid monosodium salt trihydrate with a content varying between 0.65% and 9.14% w/w, a stable combination that consist of Mannitol as diluting agent and Maltodextrin as binding agent with a content varying between 41.36% and 49.85% w/w and 49.5% w/w excipients, that consist of Sugar and Saccharin Sodium, as sweetening agents and Flavour as taste improving agent.

7. A pharmaceutical composition, according to Claim 6, consisting of: Alendronic Acid monosodium salt trihydrate, between 0.65% and 9.14% w/w and Mannitol between 40.56% and 49.05% w/w.

8. A pharmaceutical composition, according to Claim 6, consisting of: Sugar with a content of 45.00% w/w, Saccharin Sodium with a content of 0.5% w/w, Maltodextrin with a content of 0.8% w/w, Flavour with a content of 4% w/w.

9. A sachet which contains granules or powders which are prepared according to the pharmaceutical composition of claim 6.

10. A method for the preparation of granules or powders according to claim 6 which correspond to a dose and contain Alendronic Acid in the form of a monosodium salt trihydrate, acting as active ingredient. This method consisting of the following:
• The formation of a mixture of excipients (diluting agent, sweetening agents, binding agent)
• Wet granulation of the mixture with the addition of purified water
• Drying and grinding of the granules so that a mixture derives with a uniform particle size distribution
• Mixing of the deriving powder mixture with the active ingredient and the flavour,
• Filling of the sachets with the mixture of granules or powders with a quantity that corresponds to one dose.

## Patentansprüche

1. Eine pharmazeutische Zusammenstellung zur Darreichung per Os, die Alendronsäure in Form von Alendronat Natrium Trihydrat und einen pharmazeutisch akzeptablen Träger enthält, die aus einer bestimmten, konstanten Zusammensetzung von einem Verdünnungs- und einem Bindemittel besteht, wobei Mannitol als Verdünnungsmittel und prägelatinisierte Stärke oder Maltodextrin als Bindemittel und je nach pharmakotechnischer Form Natrium-Stärke-Glykolat als Dispersionsmittel, Talkum oder Magnesium Stearat als Schmiermittel, Zucker und Saccharin natrium als Süßmittel und Duftstoff als Geschmacksverbesserer zum Verbrauch kommen.

2. Eine pharmazeutische Zusammenstellung gemäß Anspruch 1, für feste Darreichungsformen per Os wie Tabletten und Kapseln, die zwischen 3,5% und 24,7% nach Gewicht Alendronat Natrium Trihydrat, zwischen 69,7% un 88.5% nach Gewicht von der konstanten Zusammensetzung bestehend aus Mannitol als Verdünnungsmittel und prägelatinisierter Stärke als Bindemittel und zwischen 5,6% und 8,0% nach Gewicht aus Hilfsstoffen bestehend aus Natrium-Stärke-Glykolat als Dispersionsmittel und Talkum oder Magnesium Stearat als Schmiermittel.

3. Eine pharmazeutische Zusammenstellung gemäß Anspruch 2, die enthält: Alendronat Natrium Trihydrat zwischen 3,5% und 24,7% nach Gewicht und Mannitol zwischen 30,6% und 39,3% nach Gewicht.

4. Eine pharmazeutische Zusammenstellung gemäß Anspruch 2, die enthält: prägelatinisierte Stärke zwischen 39,1% und 49,1% nach Gewicht, Natrium-Stärke-Glykolat zwischen 4,8% und 6,9% nach Gewicht, Talkum oder Magnesium Stearat zwischen 0,8% und 1,2% nach Gewicht.

5. Ein Tablette oder Kapsel, welche die pharmazeutische Zusammenstellung vom Anspruch 2 enthält.

6. Eine pharmazeutische Zusammenstellung gemäß Anspruch 1 für Formen, die trinkbar nach Rekonstituierung sind, wie Granulate und Pulver für trinkbare Suspension, die Alendronat Natrium Trihydrat mit einem Gehalt schwankend zwischen 0,65% und 9,14% nach Gewicht, einer konstanten Zusammemensetzung bestehend aus Mannitol als verdünnungsmittel, und Maltodextrin als Bindemittel mit einem Gehalt schwankend zwischen 41,36% und 49,85% nach Gewicht und 49,5% nach Gewicht Hilfsstoffen, bestehend aus Zucker und Saccharin natrium als Süßmittel und Duftstoff als Geschmacksverbesserer.

7. Eine pharmazeutische Zusammenstellung gemäß Anspruch 6, bestehend aus Alendronat Natrium Trihydrat zwischen 0,65% und 9,14% nach Gewicht und Mannitol zwischen 40,56% und 49,05% nach Gewicht.

8. Eine pharmazeutische Zusammenstellung gemäß Anpruch 6, bestehend aus Zucker mit einem Gehalt von 45,00% nach Gewicht, Saccharin natrium mit einem Gehalt von 0,5% nach Gewicht, Maltodextrin mit einem Gehalt von 0,8% nach Gewicht, Duftstoff mit einem Gehalt von 4% nach Gewicht.

9. Ein Beutel mit Granulat oder Pulver, die gemäß den pharmazeutischen Zusammenstellung von Anspruch 6 präpariert sind.

10. Ein Verfahren zur Präparierung von Granulaten oder Pulvern gemäß Anspruch 6, die einer Dosis entsprechen und Alendronsäure in Form von Alendronat Natrium Trihydrat als Wirkstoff enthalten. Das Verfahren besteht aus den folgenden:
a. Bildung eines Gemisches aus Hilfsstoffen (Verdünnungsmittel, Sußstoffe, Bindemittel)
b. Nasse Granulierung des Gemisches bei Zusatz von gereinigtem Wasser
c. Trocknen und Mahlen des Granulats mit einem geeignetem Sieb, damit ein Gemisch mit gleichmäßiger Partikelgröße entsteht
d. Vermischung des entstehenden Pulvergemisches mit dem Wirkstoff und dem Duftstoff
e. Abfüllung der Beutel mit dem Granulat- oder Pulvergemisch in der Menge, die einer Dosis entspricht.

## Revendications

1. Une composition pharmaceutique pour l'administration par la bouche, laquelle contient de l'Acide alendronique sel monosodique trihydraté et un pharmaceutique acceptable porteur, composée d' une combinaison précise, stable d'un moyen de dilution et d'un moyen d'une liaison en utilisant du Mannitol comme moyen de dilution et du Amidon prégélatinisé ou de la Maltodextrine comme moyen de liaison et selon la forme pharmaceutique du Sodium carboxyméthylamidon comme moyen de la désagrégation, du Talc ou du Magnésium stéarate comme agent de fertilisation, du sucre et de la Saccharine de sodium comme agents édulcorants et d' arôme pour 1' amélioration du goût.

2. Une composition pharmaceutique selon la revendication 1, pour des formes solides administrées par la bouche, telles que comprimés et gélules laquelle comprend entre 3,5% et 24,7% p/p d' Acide alendronique sel monosodique trihydraté, entre 69,7% et 88,5% p/p de la combinaison fixe, composée de Mannitol comme moyen de dilution et du Amidon prégélatinisé comme moyen de liaison, et entre 5,6% et 8,0% p/p des excipients qui consistent en Sodium carboxyméthylamidon comme moyen de désagrégation et du Talc ou du Magnésium stéarate comme agent de fertilisation.

3. Une composition pharmaceutique selon la revendication 2, laquelle comprend: De l'Acide alendronique sel monosodique trihydraté entre 3,5% et 24,7% p/p et du Mannitol entre 30,6% et 39,3% p/p

4. Une composition pharmaceutique, selon la revendication 2 laquelle comprend: Du Amidon prégélatinisé entre 39,1% et 49,1% p/p, du Sodium carboxyméthylamidon entre 4,8% et 6,9% p/p, du Talc ou du Magnésium Stéarate entre 0,8% et 1,2% p/p.

5. Un comprime ou une gélule qui comprend une composition pharmaceutique de la revendication 2.

6. Une composition pharmaceutique selon la revendication 1, pour des formes qui sont buvables à la suite d' une reconstitution tels que les grains et la poudre pour une suspension buvable, lesquelles comprennent de l'Acide alendronique sel monosodique trihydraté avec une teneur qui varie entre 0,65% et 9,14% p/p, une combinaison stable compose de Mannitol comme moyen de dilution et de la Maltodextrine comme moyen de liaison avec une teneur qui varie entre 41,36% et 49,85% p/p et 49,5% p/p excipients qui consistent en Sucre et en Saccharine de sodium comme agents edulcorants et d' arôme pour l' amélioration du goût.

7. Une composition pharmaceutique selon la revendication 6 composée de: Acide alendronique sel monosodique trihydraté entre 0,65% et 9,14% p/p et Mannitol entre 40,56% et 49,05% p/p.

8. Une composition pharmaceutique, selon la revendication 6, composée de: Sucre avec une teneur de 45,00% p/p, Saccharine de sodium avec une teneur de 0,5% p/p, Maltodextrine avec une teneur de 0,8% p/p, arôme avec une teneur de 4% p/p.

9. Un sachet qui contient des grains ou poudres préparés selon la composition pharmaceutique de la revendication 6.

10. Une méthode pour la préparation des grains ou poudres, selon la revendication 6, qui correspondent à une dose et contiennent de l'Acide alendronique sous forme de sel monosodique trihydraté, comme composant actif. La méthode est composée des suivants:
a. De la formation d' un mélange d' excipients (moyen de dilution, moyens édulcorants, moyens de liaison).
b. Transformation du mélange en forme des grains humides avec une addition d' eau nettoyée.
c. Séchage et broyage des grains avec un tamis approprié afin qu' un mélange résulte avec une taille uniforme des corpuscules.
d. Mélange de la mixtion des poudres qui résulte avec le composant actif et l'arôme.
e. Remplissage des sachets avec la mixtion des grains ou poudres avec la quantité correspondant à une dose.
